(19) Europäisches Patentamt
European Patent Office
Office européen des brevets

(11) Publication number: 0 077 099
A2

(12) # EUROPEAN PATENT APPLICATION

(21) Application number: 82201243.1

(22) Date of filing: 06.10.82

(51) Int. Cl.³: C 07 C 99/00, C 07 C 101/28, C 07 C 101/18, C 07 C 102/00, C 07 C 103/46

(30) Priority: 08.10.81 IT 2439781
07.05.82 IT 2113382

(43) Date of publication of application: 20.04.83 Bulletin 83/16

(84) Designated Contracting States: AT BE CH DE FR GB LI LU NL SE

(71) Applicant: ANIC S.p.A., Via Ruggero Settimo, 55, I-90139 Palermo (IT)

(72) Inventor: Fiorini, Mario, Viale Cortina d'Ampezzo 164, I-00135 Rome (IT)
Inventor: Riocci, Mario, Via Bixio 62, I-00015 Monterotondo Rome (IT)
Inventor: Giongo, Matteo, Via Nomentana 88/L, I-00013 Mentana Rome (IT)

(74) Representative: Roggero, Sergio et al, Ing. Barzanò & Zanardo Milano S.p.A. Via Borgonuovo 10, I-20121 Milano (IT)

(54) **Process for preparing amino acids and their esters.**

(57) An improved process is described for preparing amino acids with high optical yield, the process being based on an initial asymmetric hydrogenation of a suitable olefin-unsaturated prochiral or raceme compound, for example alpha-acetamido cinnamic acid in the case of L-N,acetylphenylalanine, in the presence of a catalyst constituted by a compound of a transition metal with asymmetric aminophosphines, in particular a rhodium complex. The hydrogenation reaction is followed by treating the final product with a solvent mixture constituted by an alcohol and a halogenated hydrocarbon, which favours the removal of the catalyst residue and simultaneously increases the optical yield of the required amino acid. The invention also provides for the preparation of amino acid esters from the corresponding N-acylamino acids without the intermediate separation of the relative amino acid. The transformation takes place in a single step which comprises the deacylation of the amino group and the esterification of the carboxyl function, in the presence of a catalyst.

EP 0 077 099 A2

This invention relates to an improved process for preparing amino acids or their derivatives, the process comprising an initial asymmetric hydrogenation of prochiral or raceme olefin-unsaturated compounds in the presence of a catalyst constituted by a compound of a transition metal with asymmetric aminophosphines, followed by treating the final product with a suitable solvent mixture constituted by an alcohol and a halogenated hydrocarbon.

The industrial preparation of optically active organic compounds with a high degree of optical purity, such as levorotatory amino acids, is still almost exclusively carried out by biochemical or microbiological processes. Up to a few years ago, no purely chemical methods were known which in terms of process economy and optical efficiency could complete with the aforesaid methods, these latter demanding rigorous procedures and careful monitoring in relation to the final purification.

However, the discovery of new homogeneous catalytic systems of high stereospecificity and the considerable developments in the synthesis of asymmetric phosphines have led to the preparation of chiral transition metal complexes having good stereoselectivity in the hydrogenation of prochiral olefins. For example, U.K. Patent Nr. 1.580.461 granted on February 11, 1981 describes a new class of optically active compounds which are very useful in the asymmetric hydrogenation of unsaturated compounds.

The said application relates to a particular class of amino derivatives of phosphorus of formula $PR^1_x(NR^2R^3)_{3-x}$ in which $R^1$ is alkyl, arylthio, alkylphosphino, arylphosphino, aminophosphino or the like; x varies from 0 to 2; and $NR^2R^3$ are optically active groups deriving from amino compounds. These compounds constitute a large category of mono and polydentate binders able to coordinate transition metals and thus form compounds capable of asymmetrically hydrogenating prochiral or raceme olefins; suitable transition metals include Ru, Rh, Pt, Mo, Fe, Ti and V amongst others.

According to this patent application, the hydrogenation reaction is carried out at a molar ratio of substrate to catalyst which varies between 10,000 and 10, and at a temperature of between -70 and +200°C.

The applicant has now discovered that which constitutes the subject matter of the present invention, namely that it is possibly to considerably improve the optical yield of the aforesaid asymmetric hydrogenation reactions if this reaction is followed by special treatment of the obtained product with a suitable solvent mixture which, in addition to favouring the removal of any catalyst residues, leads to a considerable increase in the optical efficiency of the preceding hydrogenation reaction.

The present invention therefore firstly provides a process for preparing optically active amino acids, consisting of a preliminary asymmetric hydrogenation of prochiral and raceme olefin-unsaturated compounds in the presence of a catalytic

system constituted by a transition metal compound, followed by treating the obtained product with a mixture comprising at least one alcohol and at least one halogenated hydrocarbon. Any transition metal compound known for this purpose can be used for carrying out the preliminary hydrogenation, even though the use of compounds described in the aforesaid patent has proved particularly advantageous. Likewise, said process is valid for preparing every type of amino acid and its respective derivatives, but because of the great economical and commercial interest, the applicant has considered it useful to stress the preparation of L-phenylalanine and its derivatives. It will however be simple for the expert of the art to extrapolate the necessary conditions for preparing other amino acids, and the present invention is applicable to all the different initial substrates concerned. If amino acid derivatives are to be prepared, the amino acid can be easily obtained by well known routine reactions. Thus, as stated the asymmetric hydrogenation reaction is carried out in the presence of a transition metal compound known for this purpose. The reaction is carried out in particular in the presence of a complex of a transition metal with asymmetric aminophosphines chosen from those satisfying the formula

$$PR^1_x(NR^2R^3)_{3-x}$$

in which $R^1$ is an alkyl, aryl, alkylaryl, cycloalkyl, alkoxy, aryloxy, alkylthio, arylthio, alkylphosphino or aminophosphino group; x varies from 0 to 2; $R^2$ and $R^3$, which can be the

same or different, can be an alkyl, aryl, alkylaryl, arylalkyl or cycloalkyl radical, at least one of the two containing one or more non-raceme chiral centres; in the particular case of phenylalanine, the use of rhodium complexes has proved advantageous.

The hydrogenation reaction is carried out in an alcoholic solvent at a temperature of between $-70^{\circ}C$ and $+200^{\circ}C$, with a molar unsaturated substrate:catalytic complex ratio exceeding 10, and variable up to and well beyond 20,000.

The reaction mixture obtained in this manner, and comprising the required product, the solvent and catalyst, can be treated so as to separate the product, this latter then being treated with a special solvent mixture consisting of at least one alcohol and one hydrocarbon, or alternatively this latter can be added to the alcoholic mixture originating from the introductory asymmetric hydrogenation reaction.

To obtain the required effects, i.e. removal of the catalyst residue which is as complete as possible together with increase in the optical yield, the ratio of the alcoholic component in the mixture to the hydrocarbon component must be kept between 1:1 and 1:50, the optimum ratio being related to the concentration of the raceme substance.

The present invention also provides a method for optically enriching a given amino acid, comprising treating this latter with a solvent mixture consisting of at least one alcohol and one halogenated hydrocarbon in a molar ratio varying from 1:1 to 1:50, refluxing this mixture, cooling it, then

filtering off the solid product and washing it with the halogenated hydrocarbon. Alcohols which can be used include all low-boiling alcohols, particularly ethyl alcohol, and the halogenated hydrocarbon can be advantageously chosen from those having a low number of carbon atoms, such as chloroform or methylene chloride.

The applicant has also found that amino acid esters can be prepared from the corresponding N-acylamino acids in a single stage without previously separating the intermediate. The direct transformation which is possible in this way comprises deacylation of the amino group and esterification of the carboxyl function, with considerable advantages both from the operational and from the overall yield aspects.

The general scheme is as follows:

A) 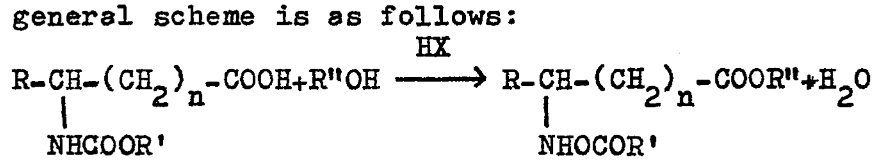


B) 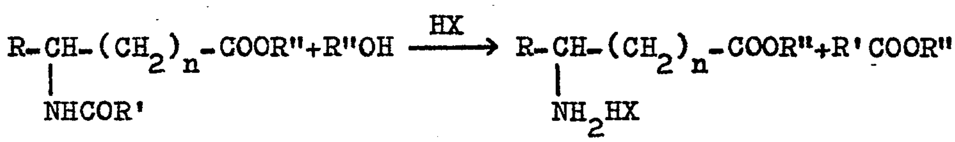


where R = R' = R" = H, alkyl, aryl, alkylaryl etc.

n = 0, 1, 2, 3 etc.

HX = Bronsted or Lewis acid.

The reaction is carried out in the presence of an acid catalyst, and from the reaction scheme it is apparent that the alcohol in the presence of the acid catalyst performs the double role of esterifying agent (reaction A) and solvolysis agent (reaction B).

The entire method of operation will be apparent from the

following examples, which however in no way limit the scope of the present invention, which is of completely general application.

EXAMPLE 1

The synthesis of L-phenylalanine will be described, this taking place by the following series of reactions

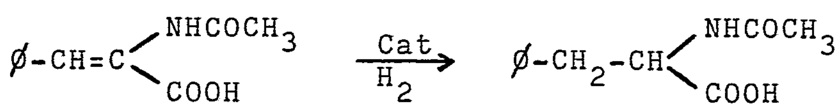


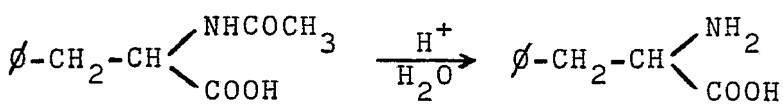


The catalyst used in the hydrogenation stage is

$[(RhPNNP\ Diene)]^+X^-$

where

Rh is rhodium

PNNP is N,N'-bis-R(+)α-methylbenzyl-N,N'-bis-diphenylphosphino-ethylenediamine

$X^-$ can be $ClO_4^-$, $PF_6^-$, or $BF_4^-$

Diene is norbornadiene (NBA) or cyclooctadiene (COD).

a) Preparation of alpha-acetamidocinnamic acid

A mixture consisting of 58.5 g (0.5 moles) of acetylglycine, 30 g (0.37 moles) of anhydrous sodium acetate, 79 g (0.74 moles) of freshly distilled benzaldehyde, and 134 g (1.25

moles) of 95% acetic anhydride are dissolved in a suitable flask of 1 litre capacity. The solution obtained is boiled under reflux for one hour, then cooled and kept at low temperature overnight.

The solid mass of yellow crystals is treated with 125 cc of cold water and ground with a stirring rod. The crystals are then transferred to a Buchner funnel and washed with cold water. After drying under vacuum over phosphorus pentoxide and potassium hydroxide, the crude azolactone weighs 69-72 g (74-77% of the theoretical). The product melts at 148-150°C, being thus sufficiently pure for preparation purposes.

47 g (0.25 moles) of the acetoaminocinnamic acid azolactone are dissolved in a round bottom short neck 1 litre flask by boiling with a mixture of 450 cc of acetone and 175 cc of water.

Hydrolysis is completed by boiling under reflux for four hours. Most of the acetone is then removed by distillation under normal pressure over a steam bath. The residual solution is diluted with 400 cc of water, heated to boiling for five minutes to ensure complete dissolving of the acetoamino acid, and then filtered. Small quantities of undissolved material (0.2 - 0.5g) on the filter are washed with 50-75 cc of boiling water. The crystals which separate from the filtrate are again dissolved by heating, after which the solution is boiled for five minutes with 10 g of norite and filtered by suction while still close to boiling point.

The norite is washed through the filter with a number of 50 cc portions (from 2 to 4) of boiling water in order to remove the

crystals which separate during filtration, and the portions of wash water are added to the filtrate. After one night at low temperature, the colourless crystalline needles are collected on a Buchner funnel, washed with 150-200 cc of iced water and dried for several hours at 90-100°C. The yield is 41-46 g (80-90% of the theoretical) of a practically pure compound, having a melting point of 191-192°C.

The reactions involved are as follows

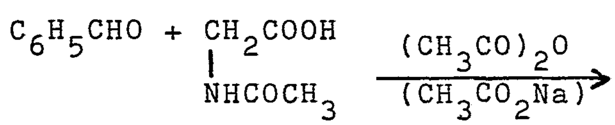


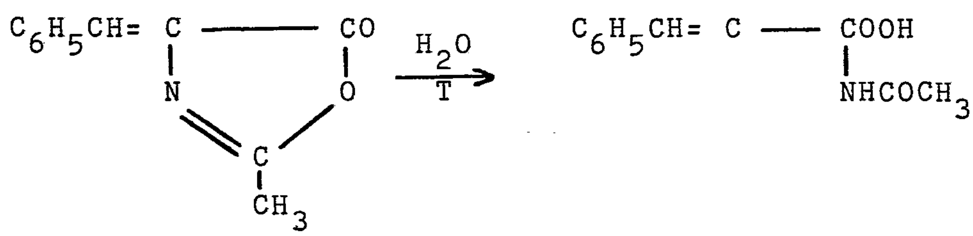


b) <u>Preparation of PNNP</u>

N,N'-bis-(R(+)alpha-methylbenzyl)-ethylenediamine is prepared from R(+)alpha-methylbenzylamine and diethyloxalate. The diamide is reduced with lithium aluminium hydride in THF, and the corresponding diamine is isolated as the dihydrochloride, M.P. 250°C (yield 80%).

After unblocking the dihydrochloride with 10% NaOH, 0.050 moles of the diamine are treated with 0.100 moles of diphenyl-

chlorophosphine in 300 ml of anhydrous benzene in the presence of 0.200 moles of triethylamine.

The mixture is heated under reflux for 20 hours, the triethylammonium hydrochloride is then separated by filtration, and the benzene solution concentrated until the N,N'-bis-(R(+) alpha-methylbenzyl)-N,N'-bis-(diphenylphosphino)-ethylenediamine separates, this having a M.P. of 138-140°C (yield 70% with respect to the initial diamine)

$$[\alpha]_D^{25} = -91.5^\circ (c = 1, CHCl_3)$$

c) Catalyst synthesis

$$PNNP + \frac{1}{2}(RhNBDCl)_2 + KPF_6 \longrightarrow (RhNBDPNNP)^+ PF_6^- + KCl$$

| | | |
|---|---|---|
| 2.096 g | 759 mg | 606 mg |
| MW 230.5 | MW 636 | MW 184 |

2.096 g of phosphine are dissolved in 25 ml of benzene in a flask, the solid Rh complex is then added, followed by the potassium salt dissolved in 6 ml of THF.

The mixture is allowed to react for 30 minutes after which 60 ml of $Et_2O$ are added. The precipitate is filtered, and after drying is washed with 10 ml of $CH_2Cl_2$, which dissolve the catalyst, the KCl being eliminated. 35 ml of $Et_2O$ are added to this solution, to precipitate the catalyst. 2.3 g of the catalyst are recovered on filtration.

d) Asymmetric hydrogenation

The hydrogenation of the alpha-acetaminocinnamic acid is carried out in an AISI 361 autoclave fitted with a mechanical stirrer, under the following conditions:

acetaminocinnamic acid 41 g (0.2 moles),

(Rh PNNP norbornadiene)$^+PF_6^-$ 0.013 g (0.133 x $10^{-4}$ moles), anhydrous ethanol 200 ml, $H_2$ pressure 1.5 atm, temperature 22°C,

substrate:catalyst ratio 15,000:1.

The autoclave is freed of oxygen by applying vacuum and then $N_2$ three or four times. Having done this, the reaction mixture suspension (catalyst + substrate) is then syphoned in. After applying vacuum and then $H_2$ twice, mechanical stirring is carried out when the operating pressure (8.5 atm) is reached.

The $H_2$ absorption kinetics and thus the conversion are as follows:

| Time | Conversion % |
|---|---|
| 15 | 39 |
| 30 | 60 |
| 45 | 75 |
| 60 | 81 |
| 75 | 86 |
| 90 | 89 |
| 120 | 95 |
| 180 | 100 |

When the reaction has terminated and the $H_2$ removed, the reaction mixture is evaporated to dryness firstly by rotavapor then by a mechanical pump. Product optical purity $\alpha_{read}$ = +0.39 (c=1 EtOH 96%) $\alpha_{max}$ = +0.469, cc = 83.3%.

e) Catalyst elimination and optical enrichment of the (L)N-acetylphenylalanine

e') Use of $CHCl_3$/EtOH

30 g of (L)N-acetylphenylalanine having an optical purity of 83.3% ($\alpha_{read}$=0.390, C=1 EtOH 96%) are treated with a mixture consisting of 180 cc of $CHCl_3$ and 9 cc of EtOH. The mixture is heated under reflux for 15 minutes. It is cooled to ambient temperature, filtered, the solid washed with $CHCl_3$ and dried. 24.205 g of (L)N-acetylphenylalanine are recovered, corresponding to a yield of 95%, with $\alpha_{read}$= +0.460, C=1 EtOH 96%, cc=98.3%. The quantity of Rh present is 8 ppm. As a general rule it can be stated that the concentration of the raceme in 96% EtOH must lie between 50 and 60%, preferably 55%, and the $CHCl_3$:EtOH ratio must be 95:5. The final product contains less than 8 ppm of Rh.

e'') Use of $CH_2Cl_2$/EtOH

5 g of (L)N-acetylphenylalanine having an optical purity of 83.3% are heated under reflux for 15 minutes in a mixture consisting of 60 ml of $CH_2Cl_2$ and 15 ml of 96% EtOH. The mixture is cooled to $0^oC$, the precipitate filtered off, washed with $CH_2Cl_2$ and dried. 3.735 g of (L)N-acetylphenylalanine are obtained, with $\alpha_{read}$=0.450 (C=1 EtOH 96%), cc=96.15%. The Rh content is less than 8 ppm.

Generalising, it can be stated that the raceme concentration in 96% EtOH must lie between 25 and 40%, preferably 35%, and the $CH_2Cl_2$:EtOH ratio must be 96:4.

EXAMPLE 2

50 g (0.241 moles) of N-acetylphenylalanine of optical purity 97% are dissolved in 400 ml of anhydrous methanol. The

resultant solution is cooled to 0°C with ice and treated with 30 g of anhydrous hydrochloric acid gas. 29.4 g (0.277 moles) of acetone dimethyl acetal are then added to the solution, and the resultant solution heated under reflux in a fractional distillation apparatus. The solution is kept at temperature for 20 hours, during which 70-80 ml of the solvent are removed periodically by fractional distillation. In this manner, the methyl ester of the acetic acid which forms as a result of reaction B is removed from the reaction medium, as is the acetone resulting from the reaction between acetone dimethyl acetal and the water which forms as a result of reaction A. In this manner, the equilibrium of both reactions is advantageously displaced in the required direction.

The L-phenylalanine hydrochloride is isolated by evaporating the solvent, and the incorporated hydrochloric acid is removed by dissolving plus evaporation carried out four times with 50 ml portions of methanol each time. In this manner 47.8 g of L-phenylalanine methyl ester hydrochloride are obtained with an optical purity equal to that of the starting substance. Yield 93%.

EXAMPLE 3

31.5 g (0.241 moles) of N-acetyl-L-alanine are reacted with 300 ml of anhydrous ethanol and 25 g of HCl gas in the manner described in example 1, but without adding acetone dimethyl acetal.

In this case, both the water formed in accordance with

reaction A and the ethyl acetate formed in accordance with reaction B are directly removed as such as a result of the formation of the ternary azeotrope with ethanol.
In this manner, 33.3 g of L-alanine ethyl ester hydrochloride are obtained with a yield of 90%.

EXAMPLE 4

28 g (0.24 moles) of N-acetylglycine in 250 ml of anhydrous methanol are reacted with 25 g of anhydrous HCl gas in the manner described in example 1. 27 g of glycine methyl ester hydrochloride are obtained, with a yield of 89%.

EXAMPLE 5

100 g (0.843 mol) of N-acetylphenylalanine having an optical purity of 97% are dissolved in 800 mls of methanol. There are added thereto 100 g of $H_2SO_4$ (96% purity, density 1.83) and the solution is refluxed. Refluxing is continued for 25 hours during which provision is made to recover, by azeotropic distillation with methanol, the methyl ester of the acetic acid as it is formed, this operation being carried out periodically. By so doing, the reaction equilibrium B (page 2) is quantitatively shifted towards the right, whereas the water as formed by the reaction A is bound by the sulphuric acid which is present in the reaction medium. On completion of the reaction, the volume of the solution is reduced to one-sixth of its original volume. Neutralization is made with aqueous ammonia until a pH of 9 is obtained. The aqueous solution is thrice extracted with dichloroethane using a 200-ml portion each time. In the organic phase, which is cooled to 0°C with an ice bath, anh. gaseous hydrogen chloride is caused to bubble.
The hydrochloride of the L-phenylalanine which has thus been formed,

is collected on a filter, washed with bichloroethane and dried. There are obtained 92 g of product, the optical yield being 99%. The yield is 89% of the theory.

06.10.82

0077099

CASE 1441/1496

CLAIMS:

1. A process for preparing optically active amino acids or their derivatives, consisting of a preliminary asymmetric hydrogenation of a prochiral or raceme olefin-unsaturated compound in the presence of a transition metal compound, followed by treating the final product with a solvent mixture comprising at least one alcohol and one halogenated hydrocarbon.

2. A process as claimed in the preceding claim, characterised in that the asymmetric hydrogenation is carried out in the presence of a complex of a transition metal with asymmetric aminophosphines chosen from those satisfying the formula

$$PR'_x(NR^2R^3)_{3-x}$$

in which $R^1$ is an alkyl, aryl, alkylaryl, cycloalkyl, alkoxy, aryloxy, alkylthio, arylthio, alkylphosphino, arylphosphino or aminophosphino group; x varies from 0 to 2; $R^2$ and $R^3$, which are the same or different, are an alkyl, aryl, alkylaryl, arylalkyl or cycloalkyl radical, at least one of the two containing one or more non-raceme chiral centres.

3. A process as claimed in the preceding claim, wherein the transition metal complex is a rhodium complex.

4. A process as claimed in claim 1, wherein the hydrogenation is carried out at a temperature between -70 and +200°C.

5. A process as claimed in claim 1, wherein the hydrogenation is carried out in the presence of an alcoholic solvent.

6. A process as claimed in claim 1, wherein the hydrogenation is carried out with the molar ratio of the unsaturated

substrate to the catalytic complex greater than 10.

7. A process as claimed in the preceding claim, wherein the molar ratio of the unsaturated substrate to the catalytic complex lies between 10 and 20,000.

8. A process as claimed in claim 1, characterised in that the asymmetric hydrogenation product is brought into contact with a mixture comprising at least one alcohol and at least one halogenated hydrocarbon in quantities such that the ratio of the alcoholic component to the hydrocarbon component lies between 1:1 and 1:50.

9. A process as claimed in the preceding claim, wherein the alcohol is ethyl alcohol.

10. A process as claimed in claim 8, wherein the halogenated hydrocarbon is chloroform or methylene chloride.

11. A process as claimed in claim 1, wherein the initial unsaturated substrate is alpha-acetamidocinnamic acid.

12. A method for increasing the optical purity of an amino acid, consisting of treating this latter with the mixture defined in claims 8 to 10.

13. A process for preparing amino acid esters, consisting of reacting the corresponding N-acylamino acid with an alcohol in the presence of an acid.